# EUROPÄISCHE PATENTANMELDUNG

(11) **EP 1 758 039 A1**
(43) Veröffentlichungstag der Anmeldung: **28.02.2007**
(21) Anmeldenummer: 05018651.9
(22) Anmeldetag: 27.08.2005
(51) Int. Cl.: G06F 19/00

(54) **Kommunikations-Adapter für ambulante medizinische oder therapeutische Geräte**

(71) Anmelder: Roche Diagnostics GmbH, 68305 Mannheim (DE); F.HOFFMANN-LA ROCHE AG, 4070 Basel (CH)
(72) Erfinder: Koehler, Matthias, 69514 Laudenbach (DE); Blasberg, Peter, 69469 Weinheim (DE); Handwerker, Guenter, 67459 Böhl-Iggelheim (DE); Aigner, Manfred, 85551 Heimstetten (DE); Habermann, Christian, 85778 Haimhausen (DE)
(74) Vertreter: Jany, Peter

(57) **Zusammenfassung**

Die Erfindung betrifft einen Kommunikations-Adapter 6 zur Verwendung mit einem ambulanten medizinischen Gerät 1. Das Gerät 1 führt eine Datenübertragung, bevorzugt eine Infrarot-Datenübertragung 5 zu dem Kommunikations-Adapter 6 durch, der über eine Datenverbindung, bevorzugt eine drahtgebundene Verbindung 10 eine Datenübertragung 8 zu einem Computer 12 durchführt. Der Kommunikations-Adapter 6 bereitet die von den Geräten 1 auszulesenden Daten derart auf, beispielsweise als HTML-Berichte, daß sie ohne spezielle Software auf einem Computer 12 dargestellt werden können, beispielsweise mit einem gebräuchlichen Internet-Browser.

## Beschreibung

Die Erfindung betrifft einen Kommunikations-Adapter zur Verwendung mit einem tragbaren, ambulant betreibbaren medizinischen oder therapeutischen Gerät, insbesondere einem Gerät für die Diagnose oder Behandlung einer Blutglukosebehandlung. Ein solcher Kommunikations-Adapter dient zum Übertragen von Daten zwischen dem medizinischen oder therapeutischen Gerät und einem Computer, der zum Anzeigen von Betriebsparametern oder Meßdaten des Geräts und/oder zum Bedienen des Geräts dient.

Ein solcher Kommunikations-Adapter wird zwischen das medizinische oder therapeutische Gerät und den Computer geschaltet und kann Daten von dem Gerät zu dem Computer übertragen, um Betriebsparameter oder Meßdaten des Geräts mit Hilfe des Computers darzustellen. Wenn der Kommunikations-Adapter auch zum Übertragen von Daten von dem Computer zu dem medizinischen oder therapeutischen Gerät ausgebildet ist, kann das Gerät auch mittels des Computers bedient werden, beispielsweise um das Gerät zu konfigurieren oder bestimmte Aktionen des Geräts auszulösen.

Das medizinische oder therapeutische Gerät umfaßt dementsprechend einen Geräteprozessor zum Steuern des Gerätes und eine Geräte-Adapter-Schnittstelle zur Kommunikation des Geräteprozessors mit dem Kommunikations-Adapter. Der Kommunikations-Adapter umfaßt einen Adapterprozessor zum Steuern des Kommunikations-Adapters, eine Adapter-Geräte-Schnittstelle zur Kommunikation des Kommunikations-Adapters mit dem Gerät, eine Adapter-Computer-Schnittstelle zur Kommunikation des Adapterprozessor mit einer Computer-Schnittstelle des Computers und einen Gerätetreiber mit zugehörigem Übertragungsprotokoll für die Kommunikation mit dem Gerät.

Die Geräte, die mit einem erfindungsgemäßen Kommunikations-Adapter Daten mit einem Computer austauschen können, sind in der Regel netzunabhängig betreibbar. Die Erfindung richtet sich insbesondere auf Geräte für die Diagnose oder Behandlung einer Erkrankung, insbesondere ein Blutglukoseerkrankung wie Diabetes. Sie richtet sich also auf analytische Meßgeräte zum Bestimmen oder Aufzeichnen (sogenannte "elektronische Tagebücher") eines medizinisch bedeutsamen Parameters, insbesondere ein Blutglukosemeter, ein Cholesterinmeßgerät, ein Blutgerinnungsparameter-Meßgerät, ein Blutdruckmeßgerät, ein Gerät zum Messen des Körpergewichts, ein Blutglukoserecorder oder ein Blutdruckrecorder, oder auf Geräte zum Injizieren eines medizinischen Wirkstoffs in einen Körper, insbesondere eine Insulinpumpe.

Ein Blutglukosemeter ist ein Blutglukose-Meßgerät, mit dessen Hilfe der Blutzuckergehalt bestimmt werden kann. In der Regel wird hierzu in einem Körper eine Einstichwunde erzeugt, ein Bluttropfen entnommen und mit Hilfe des Blutzuckermeßgeräts der Blutglukosegehalt in dem Tropfen bestimmt. Es ist aber auch denkbar, durch eine permanente Messung, beispielsweise mit in den Körper eingeführten Sensoren oder durch die Haut hindurch die Blutglukose zu messen.

Insulinpumpen sind kleine Infusionsgeräte, die am Körper getragen werden und die über einen Katheter und einer unter der Haut liegenden Nadel dem Körper kontinuierlich oder in Intervallen Insulin zuführen. Dabei kann die Dosierung auch einem bestimmten Tagesrhythmus oder bestimmten Ereignissen, wie beispielsweise der Einnahme von Mahlzeiten, angepaßt werden.

Blutglukoserecorder sind Geräte, die über eine vorgegebene Zeitdauer Blutglukosekonzentrationen aufzeichnen, beispielsweise um für einen Diabetes-Patienten ein geeignetes Insulindosierungsschema festlegen zu können.

Im folgenden wird die Erfindung ohne Beschränkung der Allgemeinheit anhand derartiger Geräte für die Diagnose oder Behandlung einer Blutglukoseerkrankung beschrieben.

Aus dem Dokument WO 98/59487 ist ein Kommunikations-Adapter bekannt, der über eine Infrarot-Schnittstelle Daten von einem medizinischen Gerät empfängt und über das Internet an eine zentrale Datenbank sendet. Hierzu wird der Kommunikations-Adapter an die Tastaturschnittstelle eines Computers mit Internet-Anschluß angeschlossen. Der Kommunikations-Adapter wandelt die von dem Gerät empfangenen Daten in Tastenbefehle um, so daß der Computer die Daten an eine zentrale Datenbank sendet. Von der zentralen Datenbank werden die empfangenen Daten aufbereitet und an den Computer, an dem der Kommunikations-Adapter angeschlossen ist, zurückgesendet und mit Hilfe eines Internet-Browsers dargestellt. Nachteilig bei diesem System ist, daß der Benutzer die Tastatur von dem Computer trennen muß, so daß der Computer nicht mehr bedient werden kann, während der Kommunikations-Adapter angeschlossen ist. Ein weiterer Nachteil ist, daß die mittels des Kommunikations-Adapters aus dem Gerät ausgelesenen Daten nur mit Hilfe eines Computers dargestellt werden können, der über einen Internet-Anschluß verfügt, und auch nur dann, wenn die Internet-Verbindung tatsächlich besteht.

Aus dem Dokument WO 00/18449 ist ein Gestell mit integrierten Infrarot-Ports bekannt, in das ein Blutglukosemeter oder eine Insulinpumpe eingelegt werden kann, um mit dem Gestell Daten auszutauschen. Dabei muß der Bediener über einen Schalter das jeweils zu dem eingelegten Gerät passende Kommunikationsprotokoll auswählen. Die Form des Gestells und die Position der Infrarot-Ports darin müssen auf die Kontur des einzulegenden Geräts abgestimmt sein. Deshalb können nicht beliebig geformte, sondern nur bestimmte Geräte mit dem Gestell ausgelesen werden, wobei die Infrarot-Ports jeweils an den richtigen Stellen angeordnet sein müssen. Neu entwickelte Geräte, die häufig ein verändertes Design haben, sind aus mechanischen Gründen wegen der veränderten Form des neuen Gehäuses sowie einer evtl. anderen Plazierung der Infrarot-Ports nicht mit dem Gestell auslesbar. Deshalb ist das Gestell für künftige, neu entwickelte Geräte nur sehr eingeschränkt geeignet. Weiterhin ist von Nachteil, daß das Gestell jeweils von Hand auf das jeweilige Gerät eingestellt werden muß, um die Kommunikation zu ermöglichen.

In dem Dokument US 6,564,105 B2 ist ein Kommunikationsprotokoll für ein implantiertes medizinisches Gerät, beispielsweise eine Infusionspumpe beschrieben.

Das Dokument US 2003/0163088 offenbart eine Infrarot-Schnittstelle in einem Computer oder in einer externen Station zum ausschließlichen Programmieren von Insulinpumpen.

Aus dem Dokument US 2003/0032867 ist bekannt, Blutglukosemeßwerte zusammen mit einem Zeit- und einem Datums-Stempel in einem Protokoll zu speichern. Die dazu gehörigen Patientendaten werden in einem weiteren Protokoll gespeichert. Die getrennte Speicherung in verschiedenen Protokollen führt zu einem erhöhten Aufwand, wenn die Daten zusammengeführt werden, z.B. in Patientenakten im Krankenhaus oder in der Arztpraxis.

Aus der Patentfamilie zu der US provisional patent application No. 60/177,414, Medical Research Group Inc. (z.B. US 6,577,899 und US 6,635,014), ist ein medizinisches System mit einem ambulanten medizinischen Gerät wie einem Blutglukosemeter oder einer Insulinpumpe bekannt, bei dem das Gerät eine Telemetrie-Einheit aufweist, die mit einer Telemetrie-Einheit eines Kommunikationsgerätes zum Auslesen von Daten und zum Programmieren der Geräte kommuniziert.

Ausgehend von diesem Stand der Technik besteht die der Erfindung zugrundeliegende Aufgabe darin, einen Kommunikations-Adapter zum Übertragen von Daten zwischen einem medizinischen oder therapeutischen Gerät und einem Computer zu schaffen, der vielseitiger einsetzbar und von dem Benutzer einfach bedienbar ist.

Diese Aufgabe wird gemäß einem ersten erfindungsgemäßen Aspekt durch einen Kommunikations-Adapter mit den Merkmalen den Anspruchs 1 und gemäß einem zweiten erfindungsgemäßen Aspekt durch einen Kommunikations-Adapter mit den Merkmalen den Anspruchs 2 gelöst. Bevorzugte Ausgestaltungen und Weiterbildungen der Erfindung ergeben sich aus den abhängigen Ansprüchen und der nachfolgenden Beschreibung mit zugehörigen Zeichnungen.

Ein erster erfindungsgemäßer Kommunikations-Adapter zur Verwendung mit einem tragbaren, ambulant betreibbaren medizinischen oder therapeutischen Gerät, insbesondere einem Gerät für die Diagnose oder Behandlung einer Blutglukoseerkrankung, zum Übertragen von Daten zwischen dem medizinischen oder therapeutischen Gerät und einem Computer, der zum Anzeigen von Betriebsparametern oder Meßdaten des Geräts und/oder zum Bedienen des Geräts dient, wobei das medizinische oder therapeutische Gerät umfaßt
- einen Geräteprozessor zum Steuern des Gerätes und
- eine Geräte-Adapter-Schnittstelle zur Kommunikation des Geräteprozessors mit dem Kommunikations-Adapter,
   und wobei der Kommunikations-Adapter umfaßt
- einen Adapterprozessor zum Steuern des Kommunikations-Adapters,
- eine Adapter-Geräte-Schnittstelle zur Kommunikation des Kommunikations-Adapters mit dem Gerät,
- eine Adapter-Computer-Schnittstelle zur Kommunikation des Adapterprozessors mit einer Computer-Schnittstelle des Computers und
- einen Gerätetreiber mit zugehörigem Übertragungsprotokoll für die Kommunikation mit dem Gerät,
   weist also die Besonderheit auf, daß
   der Kommunikations-Adapter die erforderliche Soft- oder Firmware umfaßt, um die von dem Gerät ausgelesenen Daten unabhängig von dem Computer aufzubereiten und in einem von dem Computer mit standardmäßiger Darstellungs-Software darstellbaren Datenformat über die Adapter-Computer-Schnittstelle an den Computer zu senden.
   Wenn der Kommunikations-Adapter über die Fähigkeit, d.h. die erforderliche Soft- oder Firmware verfügt, um die Daten für die Darstellen mit einer standardmäßigen Darstellungssoftware des Computers aufzubereiten, ist an dem Computer kein besonderes zusätzliches Programm zum Darstellen der Daten erforderlich. In einer bevorzugten Ausführungsform ist das von dem Kommunikations-Adapter zur Darstellung der mit dem Computer von dem Gerät ausgelesenen Daten erzeugte Datenformat ein von einem Internet-Browser darstellbares Format, vorzugsweise ein HTML-Format.
   Die Soft- bzw. Firmware zum Auslesen der Geräte und zum Aufbereiten der Daten befindet sich in dem Kommunikations-Adapter. Dieser leitet die Daten in einem von einem Internet-Browser darstellbaren Format, z.B. als HTML-Datei an den Computer weiter. In dem Computer wird ein standardmäßiges Programm verwendet, um die Daten in Form von Graphen, Statistiken und Tabellen auf dem Monitor des Computers anzuzeigen. Ein standardmäßiges Programm in diesem Sinne ist ein Programm, das auch auf einem Computer installiert wäre, der nicht zur Benutzung mit einem erfindungsgemäßen Kommunikations-Adapter vorgesehen ist. Bevorzugte standardmäßige Programme im Rahmen der Erfindung sind Internet-Browser wie z.B. Internet Explorer, Netscape oder Mozilla.
   Ein solcher Internet-Browser ist in der Regel in dem Betriebssystem eines Computers installiert, wobei die verschiedenen Internet-Browser dasselbe Datenformat verarbeiten können, insbesondere ein HTML-Datenformat.
   Der Computer dient als temporärer Speicher für die von dem Kommunikations-Adapter empfangenen Daten bzw. HTML-Daten. Über Monitor, Maus und Tastatur des Computers wird der Kommunikations-Adapter zum Auslesen der Daten gesteuert. Der Computer dient also nur als User-Interface für das Anzeigen der Daten und das Bedienen des Kommunikations-Adapters, ohne daß hierzu eine spezielle Software auf dem Computer installiert oder gestartet werden muß. Aus Datenschutzgründen ist es vorteilhaft, daß dabei keine der von dem Gerät ausgelesenen Daten auf dem Computer gespeichert werden.
   Gemäß einem zweiten, selbstständig oder in Kombination mit dem ersten erfindungsgemäßen Aspekt nutzbaren erfindungsgemäßen Aspekt wird ein Kommunikations-Adapter zur Verwendung mit einem tragbaren, ambulant betreibbaren medizinischen oder therapeutischen Gerät, insbesondere einem Gerät für die Diagnose oder Behandlung einer Blutglukoseerkrankung, zum Übertragen von Daten zwischen dem medizinischen oder therapeutischen Gerät und einem Computer, der zum Anzeigen von Betriebsparametern oder Meßdaten des Geräts und/oder zum Bedienen des Geräts dient, wobei das medizinische oder therapeutische Gerät umfaßt
- einen Geräteprozessor zum Steuern des Gerätes und
- eine Geräte-Adapter-Schnittstelle zur Kommunikation des Geräteprozessors mit dem Kommunikations-Adapter,
   und wobei der Kommunikations-Adapter umfaßt
- einen Adapterprozessor zum Steuern des Kommunikations-Adapters,
- eine Adapter-Geräte-Schnittstelle zur Kommunikation des Kommunikations-Adapters mit dem Gerät,
- eine Adapter-Computer-Schnittstelle zur Kommunikation des Adapterprozessors mit einer Computer-Schnittstelle des Computers und
- einen Gerätetreiber mit zugehörigem Übertragungsprotokoll für die Kommunikation mit dem Gerät,
   vorgeschlagen, der die Besonderheit aufweist, daß die Steuerung des Kommunikations-Adapters von dem Computer aus dadurch erfolgt, daß von dem Computer Lesebefehle an den Kommunikations-Adapter gesendet werden, durch die in dem Kommunikations-Adapter Triggerdateien gelesen werden, die von der Soft- oder Firmware des Kommunikations-Adapters als Steuerbefehle interpretiert werden.

Das Senden der Lesebefehle von dem Computer an den Kommunikations-Adapter erfolgt dabei bevorzugt über die Computer-Schnittstelle des Computers an die Adapter-Computer-Schnittstelle des Kommunikations-Adapters.

Die Befehlssteuerung des Kommunikations-Adapters erfolgt gemäß diesem zweiten erfindungsgemäßen Aspekt über Lesebefehle (Links) auf das Speichermedium des Kommunikations-Adapters. Wenn auf dem Bildschirm ein Link angeklickt wird, werden auf dem Kommunikations-Adapter ein oder mehrere bestimmte sogenannte Triggerdateien gelesen und das Lesen einer solchen Datei wird von der Soft- oder Firmware des Kommunikations-Adapters als ein bestimmter Befehl, z.B. "Drucken" oder "Gerät lesen" interpretiert. Auf diese Weise ist es möglich, den Kommunikations-Adapter mit dem Computer zu steuern, und zwar mit einer standardmäßig auf dem Computer installierten Software wie beispielsweise einem Internet-Browser, ohne daß hierzu ein besonderes Software-Programm auf dem Computer installiert ist, das Befehle ausführen kann.

In dem bevorzugten Fall der Verwendung eines Internet-Browsers zum Darstellen der ausgelesenen Daten und zum Steuern des Kommunikations-Adapters ist ferner zu berücksichtigen, daß mit standardmäßigen Internet-Browsern keine Schreibbefehle an einen Computer gesendet werden können, um zu verhindern, daß beispielsweise beim Öffnen einer Internetseite durch solche Befehle das automatische Kopieren oder Löschen auf dem Computer vorhandener Dateien gestartet wird. Daher ist insbesondere bei der Verwendung von Internet-Browsern das Lesen von Triggerdateien zum Steuern des Kommunikations-Adapters von dem Computer aus vorteilhaft.

In der Praxis ist es vorteilhaft, wenn der Trigger für einen Befehl in dem Lesen mehrerer Dateien, bevorzugt in unterschiedlichen Verzeichnissen auf dem Kommunikations-Adapter, gleichzeitig oder in einer festen Reihenfolge nacheinander besteht. Auf diese Weise kann nicht nur der Codierungsgrad für die möglichen Befehle, sondern auch die Sicherheit erhöht werden, beispielsweise um zu vermeiden, daß beim Scannen des Kommunikations-Adapters mit einem Virenscanner oder beim Betrachten des Inhalts des Kommunikations-Adapters mit einem anderen Programm, beispielsweise einem Windows-Explorer, unbeabsichtigt Befehle an den Kommunikations-Adapter gesendet werden.

Die nachfolgend beschriebenen Merkmale sind bei jeweils beiden erfindungsgemäßen Aspekten oder bei einer Kombination der beiden erfindungsgemäßen Aspekte vorteilhaft einsetzbar.

Nach einem ersten vorteilhaften Merkmal wird vorgeschlagen, daß die Adapter-Geräte-Schnittstelle zur Kommunikation des Kommunikations-Adapters mit dem Gerät eine Schnittstelle zur drahtlosen Datenübertragung, insbesondere eine Infrarot-Schnittstelle ist. Dementsprechend ist dann auch die Geräte-Adapter-Schnittstelle zur Kommunikation des Geräteprozessors mit dem Kommunikations-Adapter eine Schnittstelle zur drahtlosen Datenübertragung, insbesondere eine Infrarot-Schnittstelle.

Prinzipiell kann die Adapter-Geräte-Schnittstelle auf jede beliebige Weise implementiert sein. Im Falle einer drahtgebundenen Datenübermittlung muß der Benutzer des Gerätes jedoch ein passendes Anschlußkabel zur Verfügung haben und anschließen, um die Kommunikation des Gerätes mit einem Kommunikations-Adapter aufzubauen. Dies kann, insbesondere bei erkrankten Personen, Schwierigkeiten bereiten. Darüber hinaus können die Steckverbindungskontakte verschmutzen oder beschädigt werden. Die drahtlose Kommunikation zwischen dem Gerät und dem Kommunikations-Adapter, beispielsweise per Funk, ist daher bevorzugt. In der Praxis ist die Implementierung in Form einer Infrarot-Schnittstelle bevorzugt, da diese leicht benutzbar und unanfällig sowie darüber hinaus kostengünstig realisierbar ist, so daß die meisten Geräte ohnehin über eine solche Infrarot-Schnittstelle verfügen.

Die Adapter-Computer-Schnittstelle zur Kommunikation des Adapterprozessors mit der Computer-Schnittstelle des Computers und dementsprechend die Computer-Schnittstelle des Computers ist dagegen bevorzugt eine Schnittstelle zur drahtgebundenen Datenübertragung. Bei gebräuchlichen Computern ist es unüblich, eine Schnittstelle für die drahtlose Kommunikation ab Werk vorzusehen, dagegen ist der Einbau von Schnittstellen für die drahtgebundene Kommunikation, beispielsweise einer USB-Schnittstelle, der Regelfall. Eine drahtlose Kommunikation zwischen Computer und Kommunikations-Adapter kann aber ebenfalls vorteilhaft sein, beispielsweise in Form einer WLAN-Verbindung.

Die Verwendung einer drahtgebundenen Schnittstelle für die Datenübertragung zwischen Kommunikations-Adapter und Computer vergrößert daher den Anwendungsbereich für einen Benutzer. Dabei ist es für den Benutzer in vielen Anwendungsfällen nicht nachteilig, daß eine Kabelverbindung zwischen Computer und Kommunikations-Adapter hergestellt werden muß, beispielsweise wenn der Kommunikations-Adapter nicht von dem Benutzer des Gerätes mitgeführt wird, beispielsweise wenn sich der Benutzer mit seinem Gerät an eine Auslesestation begibt, an der bereits ein Kommunikations-Adapter an einen Computer angeschlossen ist, wie z.B. in einer Apotheke oder in einer Arztpraxis.

Nach einem zusätzlichen bevorzugten Merkmal wird vorgeschlagen, daß der Kommunikations-Adapter eine Mehrzahl von Gerätetreibern mit zugehörigen Übertragungsprotokollen aufweist, die bei einem über die Adapter-Geräte-Schnittstelle mit dem Kommunikations-Adapter kommunizierenden Gerät automatisch abgefragt werden, um einen für das aktuelle Gerät passenden Gerätetreiber aus der Mehrzahl der Gerätetreiber zu wählen. Hierbei kann auch das Kommunikationsprotokoll zu verschiedenen Geräten unterschiedlich sein, solange die Voraussetzungen zur Nutzung derselben Hardware vorliegen. Beispielsweise können manche Geräte einen proprietären Kommunikations-Standard und andere einen allgemeinen IrDA-Standard verwenden.

Vorteilhafterweise wird das Durchprobieren der Gerätetreiber zum Aufbauen einer Kommunikation mit einem Gerät automatisch gestartet, sobald der Kommunikations-Adapter mit Strom versorgt wird. Das Durchprobieren kann auch beim Erkennen eines Gerätes fortgesetzt werden, um zu überprüfen, ob eventuell weitere Geräte vorhanden sind, mit denen ggf. gleichzeitig kommuniziert werden soll, oder das Durchprobieren kann beim Trennen einer Kommunikationsverbindung zu einem Gerät neu gestartet werden, um die Verbindung zu diesem oder einem anderen Gerät neu aufzubauen. Diese automatischen Abläufe erleichtern die Verwendung des Kommunikations-Adapters durch einen Benutzer oder insbesondere durch wechselnde Benutzer oder bei der Verwendung unterschiedlicher Geräte.

Auf diese Weise kann ein mit dem Kommunikations-Adapter auszulesendes Gerät, beispielsweise ein Blutglukosemeter, eine Insulinpumpe oder ein elektronisches Tagebuch für Blutglukosewerte, automatisch von dem Kommunikations-Adapter erkannt werden. Geräte wie die für die Diagnose oder Behandlung einer Blutglukoseerkrankung besitzen zumeist eine Infrarot-Schnittstelle für die externe Kommunikation, die jeweils verwendeten Protokolle und Befehlssätze sind jedoch unterschiedlich. Daher ist es vorteilhaft, wenn die Soft- bzw. Firmware des Kommunikations-Adapters die in Frage kommenden Gerätetreiber, die die entsprechenden Befehlssätze beinhalten, aufweist. Währen des Aufbaus der Kommunikation zwischen dem Kommunikations-Adapter und dem Gerät fragt der Kommunikations-Adapter nacheinander mit den in ihm vorhandenen Kommunikationsprotokollen (Treibern) das zunächst unbekannte Gerät an, bis er das passende Kommunikationsprotokoll gefunden hat, mit dem die Kommunikation mit dem Gerät durchführbar ist. Ab dann wird die Kommunikation mit diesem gefundenen Treiber durchgeführt.

Dabei kann vorteilhafterweise auch die Übertragungsrate (baud rate) optimiert werden. Hierzu fragt beispielsweise der Kommunikations-Adapter mit einer niedrigen Ausgangs- oder Default-Übertragungsrate bei dem Gerät an, welche Übertragungsrate das Gerät ermöglicht, und schaltet danach auf die höchste gemeinsame Übertragungsrate um.

Auf diese Weise ist es aber nicht nur möglich, ein zunächst unbekanntes Gerät zu erkennen, sondern es kann auch mit künftigen, neu entwickelten Geräten kommuniziert werden, vorausgesetzt, daß eine Infrarot-Schnittstelle verwendet wird. Ggf. muß lediglich ein neuer Softwaretreiber in dem Kommunikations-Adapter aufgespielt werden. Ein derartiges Software-Update kann beispielsweise über einen Download von einer im Internet vom Hersteller bereitgestellten Treiberdatei oder mittels einer CD erfolgen, wenn der Kommunikations-Adapter an einen Computer angeschlossen ist. Der Kommunikations-Adapter ist also updatefähig für die Kommunikation mit neuen Geräten oder mit geänderter Gerätesoftware.

Beim Aktivieren der Kommunikation des Kommunikations-Adapters mit einem Gerät kann das Suchen nach dem passenden Gerätetreiber in dem Kommunikations-Adapter dadurch optimiert werden, daß zunächst die Kommunikation mit dem zuletzt als zutreffend erkannten Gerätetreiber oder einem Gerätetreiberpaar, bestehend beispielsweise aus Blutglukosemeter und Insulinpumpe, gestartet wird oder dieser zuletzt verwendete Treiber beim Durchprobieren der Gerätetreiber häufiger als die anderen zum Testen eines Kommunikationsaufbaus eingesetzt wird. Dadurch kann bei den gebräuchlichen Anwendungen eines Benutzers mit seinen Geräten die Kommunikation zwischen dem Kommunikations-Adapter und dem Gerät schneller aufgebaut werden.

Nach einem vorteilhaften zusätzlichen Merkmal kann vorgesehen sein, daß der Kommunikations-Adapter zusätzlich die von dem Gerät ausgelesenen Daten in einem abspeicherbaren Datenformat erzeugt und diese Daten in mindestens einer Datei in dem Kommunikations-Adapter abspeichert, von wo sie über die Adapter-Computer-Schnittstelle von dem Computer auslesbar sind. Der Kommunikations-Adapter kann auch in diesem Fall in einem Stand-Alone-Modus arbeiten, erzeugt die Daten jedoch nicht nur beispielsweise als HTML-Datei für die Darstellung mit einem Internet-Browser, sondern zusätzlich z.B. als XML-Datei, die dann in eine Datenbank oder Tabelle, beispielsweise Excel, importiert werden kann.

Auf dem Computer ist hierzu eine standardmäßige Software oder eine firmenspezifische Software gespeichert, die die Dateien importieren, weiterverarbeiten und daraus die Darstellung generieren oder in einer Datenbank ablegen kann. Der Kommunikations-Adapter generiert diese Dateien selbständig, obwohl er sich eigentlich dem Computer gegenüber wie ein passives Gerät, beispielsweise wie ein USB-Gerät oder ein Massenspeichergerät wie ein Memory-Stick, darstellt. Der Kommunikations-Adapter kann den Computer durch sich Ab- und wieder Anmelden zum Neuladen des Dateisystems des Kommunikations-Adapters veranlassen und der Kommunikations-Adapter dient zusammen mit der firmenspezifischen Software als "intelligentes Kabel" zum Aufbereiten der von dem Gerät ausgelesenen Daten.

Die Erfindung wird nachfolgend anhand in den Figuren dargestellter Ausführungsbeispiele näher erläutert. Die darin beschriebenen Besonderheiten können einzeln oder in Kombination miteinander eingesetzt werden, um bevorzugte Ausgestaltungen der Erfindung zu schaffen. Es zeigen:
- Fig. 1: einen Kommunikations-Adapter zum Auslesen von Daten von Geräten,
- Fig. 2: einen Kommunikations-Adapter zum Auslesen von Daten von Geräten und zum Bedienen der Geräte von einem Computer aus und

- Fig. 3: einen schematischen Aufbau der Prozeßsteuerung eines Kommunikations-Adapters mit zugehöriger Systemumgebung.

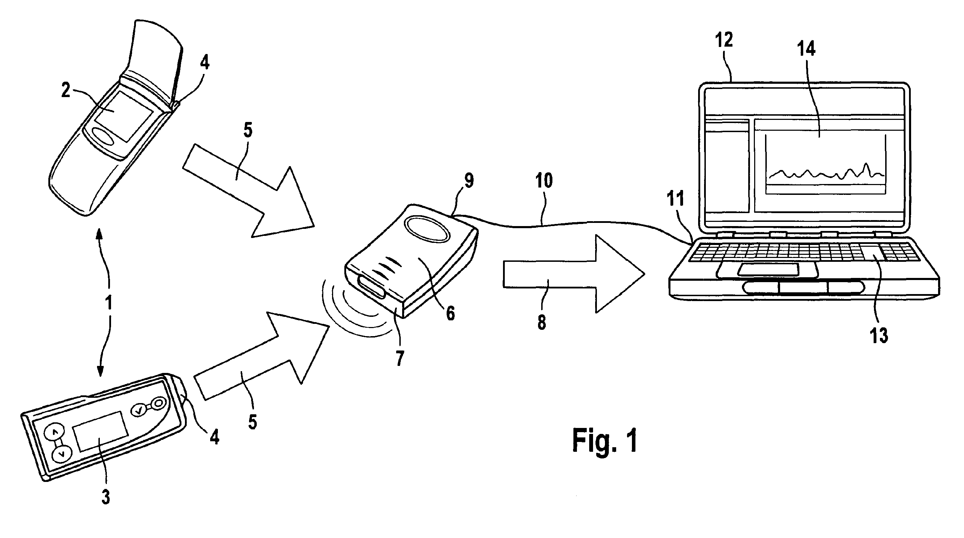

Die Fig. 1 zeigt zwei tragbare, netzunabhängig ambulant betreibbare medizinische Geräte 1, und zwar ein Blutglukosemeter 2 und eine Insulinpumpe 3, die von einem Benutzer ggf. in Kombination miteinander verwendet werden. Beide Geräte weisen eine Geräte-Adapter-Schnittstelle 4 zum Senden und/oder Empfangen von Daten auf, die als Infrarot-Schnittstelle ausgebildet ist. In dem in Fig. 1 dargestellten Ausführungsbeispiel erfolgt die Infrarot-Datenübertragung 5 durch Senden von Daten von der Geräte-Adapter-Schnittstelle 4 des Geräts 1 an einen Kommunikations-Adapter 6, der eine Adapter-Geräte-Schnittstelle 7 zum Senden und/oder Empfangen von Daten aufweist, die ebenfalls als Infrarot-Schnittstelle ausgebildet ist.

Der Kommunikations-Adapter 6 kann vorteilhafterweise als Stand-Alone-Gerät ausgebildet sein. Er ist für die drahtgebundene Datenübertragung 8 mit einer Adapter-Computer-Schnittstelle 9 und einem Übertragungskabel 10 an eine Computer-Schnittstelle 11 eines Computers 12 angeschlossen. Der Computer 12 kann beispielsweise ein PersonalComputer, ein Laptop, ein Handheld-Computer oder der Computer in einem Internet-Café, in einer Arztpraxis oder in einer Apotheke, wohin sich der Benutzer der Geräte 1 nur mit seinem Gerät 1 oder mit seinem Gerät 1 und einem Kommunikations-Adapter 6 begibt, sein.

Eine drahtgebundene Computer-Schnittstelle 11 des Computers 12 kann beispielsweise eine serielle Schnittstelle, eine parallele Schnittstelle, eine Firewire-Schnittstelle oder bevorzugt eine USB-Schnittstelle sein. Das Übertragungskabel 10 und eine Adapter-Computer-Schnittstelle 9 an dem Kommunikations-Adapter 6 werden passend dazu ausgewählt. Ggf. kann ein Kommunikations-Adapter 6 mehrere drahtgebundene Schnittstellen in verschiedenen Standards aufweisen. Die USB-Schnittstelle ist bevorzugt, weil sie sehr weit verbreitet ist, einen geringen Platzbedarf hat und eine schnelle Datenübertragung ermöglicht. Auch eine drahtlose Kommunikation zwischen Kommunikations-Adapter 6 und Computer 12 kann vorteilhaft sein, beispielsweise in Form einer WLAN-Verbindung.

Zum Auslesen der Daten der Geräte 1 und deren Darstellung auf dem Monitor 14 des Computers 12 wird zunächst der Kommunikations-Adapter 6 mit dem Kabel 10 an den Computer 12 angeschlossen. In bevorzugten Ausführungsformen weist der Kommunikations-Adapter 6 keine eigene oder interne Stromquelle auf und seine Stromversorgung erfolgt über das Kabel 10 von der Computer-Schnittstelle 11 des Computers 12. Der Vorteil für den Benutzer besteht darin, daß der Kommunikations-Adapter 6 klein und leicht sein kann, da kein eigenes Netzteil erforderlich ist, und auch keine den Platzbedarf und das Gewicht erhöhenden Akkus oder Batterien enthalten sein und mitgeführt werden müssen.

Mit dem Anschließen des Kommunikations-Adapters 6 an den Computer 12 geht der Kommunikations-Adapter 6 automatisch in den Suchmodus über, in dem er nach Geräten 1 sucht, die mit seiner Adapter-Geräte-Schnittstelle 7 kommunizieren können. Alternativ kann auch vorgesehen sein, daß der Kommunikations-Adapter 6 durch eine Aktion des Benutzers, beispielsweise das Drücken einer Taste, in den Suchmodus geschaltet wird.

Wenn die Geräte-Adapter-Schnittstelle 4 des Gerätes 1 eine Infrarot-Schnittstelle ist, sollte sie mit der Adapter-Geräte-Schnittstelle 7 des Kommunikations-Adapters 6, die dann ebenfalls eine Infrarot-Schnittstelle ist, ausgerichtet werden, um die optische Datenübertragung zu ermöglichen. Der Kommunikations-Adapter 6 scrollt die in ihm gespeicherten Gerätetreiber durch und sucht nach einem passenden Gerät 1, das mit ihm kommunizieren kann.

Die Geräte-Adapter-Schnittstelle 4 an dem Gerät 1, insbesondere eine Infrarot-Schnittstelle, wird vorteilhafterweise durch eine manuelle Betätigung des Benutzers an dem Gerät 1 aktiviert, um die Kommunikation mit dem Kommunikations-Adapter 6 aufzubauen und anschließend die Datenübertragung bzw. Infrarot-Datenübertragung 5 zu ermöglichen. Die manuelle Aktivierung der Geräte-Adapter-Schnittstelle 4 ermöglicht es, daß sie beim sonstigen Gebrauch des Geräts 1 aus Stromspar- und Datensicherheitsgründen abgeschaltet ist.

Wenn der Computer 12 den angeschlossenen Kommunikations-Adapter 6 erkannt hat und die Kommunikation des Kommunikations-Adapters 6 mit dem Gerät 1 aufgebaut ist, kann der Benutzer an der Tastatur 13 des Computers einen Internet-Browser aufrufen und die von dem Gerät 1 ausgelesenen Daten mittels des Internet-Browsers auf dem Monitor 14 darstellen, ohne daß hierzu eine besondere, spezielle oder individuelle Programmierung des Computers 12 erforderlich ist. Der Benutzer des Gerätes 1 und des Kommunikations-Adapters 6 kann daher die Daten auf jedem Computer 12, auf dem ein Internet-Browser installiert ist, darstellen und betrachten, ohne hierzu eine spezielle Software für das Auslesen des Kommunikations-Adapters 6 auf dem Computer 12 installieren zu müssen.

Dies wird dadurch ermöglicht, daß der Kommunikations-Adapter 6 die erforderliche Soft- oder Firmware umfaßt, um die von dem Gerät 1 ausgelesenen Daten unabhängig von dem Computer 12 aufzubereiten und in einem von dem Computer 12 mit standardmäßiger Darstellungs-Software darstellbaren Datenformat über die Adapter-Computer-Schnittstelle 9 an den Computer 12 zu senden. Andererseits kann auch die Steuerung des Kommunikations-Adapters 6 von dem Computer 12 aus dadurch erfolgen, daß von dem Computer 12 Lesebefehle an den Kommunikations-Adapter 6 gesendet werden, durch die in dem Kommunikations-Adapter 6 Triggerdateien gelesen werden, die von der Soft- oder Firmware des Kommunikations-Adapters 6 als Steuerbefehle interpretiert werden.

Da sich die zum Darstellen der Daten erforderliche Soft- bzw. Firmware in dem Kommunikations-Adapter 6 befindet und der Computer 12 nur als Anzeige und Bedienelement dient, das nur einen, standardmäßig im Betriebssystem vorhandenen, Internet-Browser benötigt, ist die Verwendung des Kommunikations-Adapters 6 zum Darstellen der Daten unabhängig davon, welcher Computer 12 verwendet wird. Dadurch kann der Kommunikations-Adapter 6 von dem Benutzer sehr mobil genutzt werden, da der Kommunikations-Adapter 6 beispielsweise auch auf Reisen an einen beliebigen Computer 12 anschließbar ist. Es handelt sich um ein Real Plug & Play System; man muß lediglich die Verbindung zwischen Kommunikations-Adapter 6 und Computer 12, beispielsweise eine USB-Verbindung mit dem Übertragungskabel 10, herstellen und kann die Daten der Geräte 1 auf dem Computer 12 darstellen. Hierzu sind auch keinerlei Administratorrechte notwendig, so daß der Kommunikations-Adapter 6 selbst bei Rechnern verwendet werden kann, die gegen die Installation von zusätzlicher Software durch einen Benutzer gesperrt sind.

Ein weiterer Vorteil ist, daß es sich um ein sogenanntes "Embedded System" handelt, das sehr virensicher ist. Da der Computer 12 nur als User-Interface dient und der Benutzer mit diesem User-Interface vertraut ist, da er die Bedienung eines Internet-Browsers, einer Tastatur, einer Maus und eines Monitors kennt, ist die Bedienung für den Benutzer sehr leicht erlernbar.

Die Verwendung des Computers 12 zum Darstellen der von den Geräten 1 ausgelesenen Daten hat für den Benutzer gegenüber einem Betrachten der Daten auf den Anzeigeelementen der Geräte 1 viele Vorteile. Auf den kompakten Geräten 1 befinden sich nur kleine Anzeigeelemente, die nur wenig Information in kleiner Auflösung, in kleinem Format und meist nur Einzelwerte darstellen können. Eine anspruchsvollere Darstellung oder Auswertung der Daten ist nicht oder nur sehr mühsam möglich. Auf dem Monitor 14 des Computers 12 kann dagegen eine aufwendige und aufbereitete Darstellung von Daten in Graphen oder Listen sowie eine Trendanalyse erfolgen.

Dabei kann der Benutzer sogar auch in Fällen, in denen die Datenübertragung, beispielsweise die Infrarot-Datenübertragung 5, nur von dem Gerät 1 zu dem Kommunikations-Adapter 6 und nicht von dem Kommunikations-Adapter 6 zu dem Gerät 1 erfolgt, die Darstellung auf dem Monitor 14 anpassen. Hierzu können in dem Kommunikations-Adapter 6 verschiedene, fest vorgegebene Darstellungsvarianten abgespeichert sein, die der Benutzer an dem Computer 12 alternativ abrufen kann.

Der Computer 12 kann jedoch nicht nur beispielsweise Meßdaten, Profile, Muster, Trends, Fehlverhalten und Fehldosierungen graphisch darstellen, sondern auch Geräteparameter wie beispielsweise die Seriennummer des ausgelesenen Geräts 1 oder eine Patientenidentifikation. Die ausgelesenen HTML-Daten werden, anders als ggf. die mit einer anderen Software zusätzlich oder alternativ ausgelesenen XML-Daten, beim Trennen der Adapter-Computer-Schnittstelle 9 von der Computer-Schnittstelle 11 vorzugsweise auf dem Computer 12 gelöscht, was insbesondere bei Verwendung des Kommunikations-Adapters 6 an öffentlich zugänglichen Computern 12, beispielsweise in einer Arztpraxis oder einer Apotheke, in die sich ein Benutzer mit seinem Gerät 1 begibt, um an einem dort vorhandenen Kommunikations-Adapter 6 die Daten auszulesen, die Vertraulichkeit wahrt. Alternativ besteht auch die Möglichkeit, an dem Kommunikations-Adapter 6 eine Reset-Taste oder auf dem Monitor 14 zur Bedienung des Kommunikations-Adapters 6 ein Reset-Icon vorzusehen.

Aus Gründen der Vertraulichkeit ist es ebenfalls vorteilhaft, wenn die von dem Gerät 1 ausgelesenen Daten in dem Kommunikations-Adapter 6 in einem flüchtigen Speicher gespeichert oder beim Trennen der Datenübertragungsverbindung zu einem Gerät 1, beim Anschließen eines neuen Gerätes 1 an den Kommunikations-Adapter 6, durch Drücken einer Reset-Taste an dem Kommunikations-Adapter 6 oder durch Anklicken eines Reset-Buttons auf dem Monitor 14 gelöscht werden.

In manchen Ausführungsformen kann vorgesehen sein, daß der Benutzer an dem Computer 12 auch online mit einem Web-Server oder Host-Computer kommuniziert, beispielsweise auf einer Website des Herstellers seines Gerätes 1, und dieser Web-Server mit dem Kommunikations-Adapter 6 kommuniziert, der an den Computer 12 angeschlossen ist. Auf diese Weise kann beispielsweise ein Update der Soft- oder Firmware des Kommunikations-Adapters 6 und/oder des Gerätes 1 stattfinden.

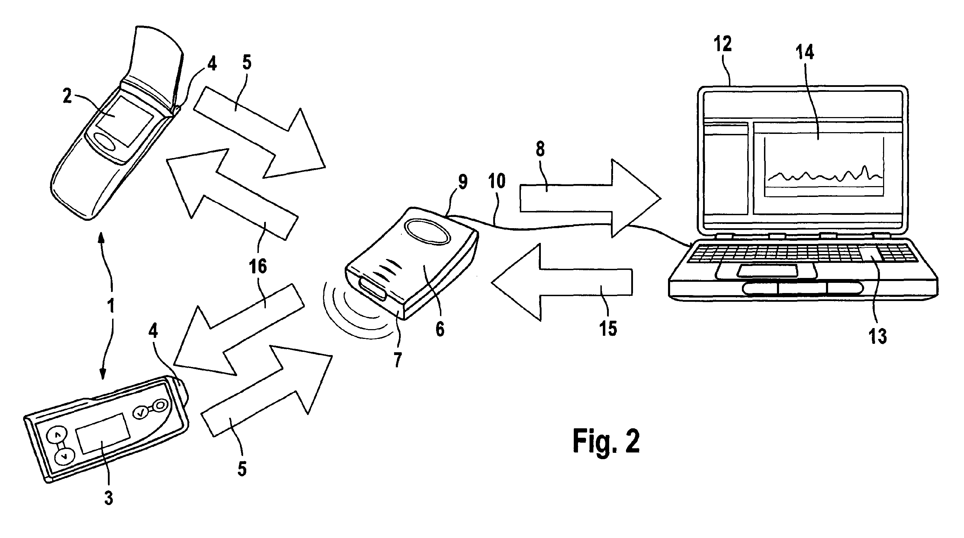

In Fig. 2 ist ein weiteres Ausführungsbeispiel dargestellt. Es entspricht demjenigen von Fig. 1, wobei eine weitere Funktionalität ausgebildet ist, nämlich die, daß der Kommunikations-Adapter 6 zum Übertragen von Daten an das Gerät 1 zum Steuern oder Programmieren des Geräts 1 von dem Computer 12 aus ausgebildet ist, wobei die Steuerung oder Programmierung des Geräts 1 von dem Computer 12 über die Computer-Schnittstelle 11 des Computers 12 an den Kommunikations-Adapter 6 und von dem Kommunikations-Adapter 6 über die Adapter-Geräte-Schnittstelle 7 des Kommunikations-Adapters 6 an das Gerät 1 erfolgt. Bei der in Fig. 2 dargestellten Ausführungsform findet also auch eine drahtgebundene Datenübertragung 15 von dem Computer 12 zu dem Kommunikations-Adapter 6 und eine Infrarot-Datenübertragung 16 von dem Kommunikations-Adapter 6 zu dem Gerät 1 statt.

In diesem Fall dient der Kommunikations-Adapter 6 sozusagen als "intelligentes Kabel" bzw. als USB-zu-IR-Adapter. Der Kommunikations-Adapter 6 setzt die Daten von dem USB-Protokoll in das IR-Protokoll und umgekehrt um. In diesem Fall kann auf dem Computer 12 eine zusätzliche spezielle Software mit den Gerätetreibern, z.B. für die Datenanalyse gespeichert werden, oder die Computer-Software nutzt die Gerätetreiber und die ausgelesenen Daten, die vom Kommunikations-Adapter 6 zur Verfügung gestellt werden. Für die tatsächliche Kommunikation zu dem Gerät 1 sorgt die Firmware in dem Kommunikations-Adapter 6. Die Daten können aus den Geräten 1 über die Datenübertragungen 5 und 8 ausgelesen und am Computer 12 dargestellt werden. Über die Datenübertragungen 15 und 16 können die Geräte 1 darüber hinaus bedient, programmiert oder eingestellt werden. Dadurch ist es beispielsweise möglich, bei einem Blutglukosemeter 2 Datum und Uhrzeit einzustellen oder bei einer Insulinpumpe 3 Datum und Uhrzeit einzustellen und ein bestimmtes zeitliches Basalratenprofil einzustellen.

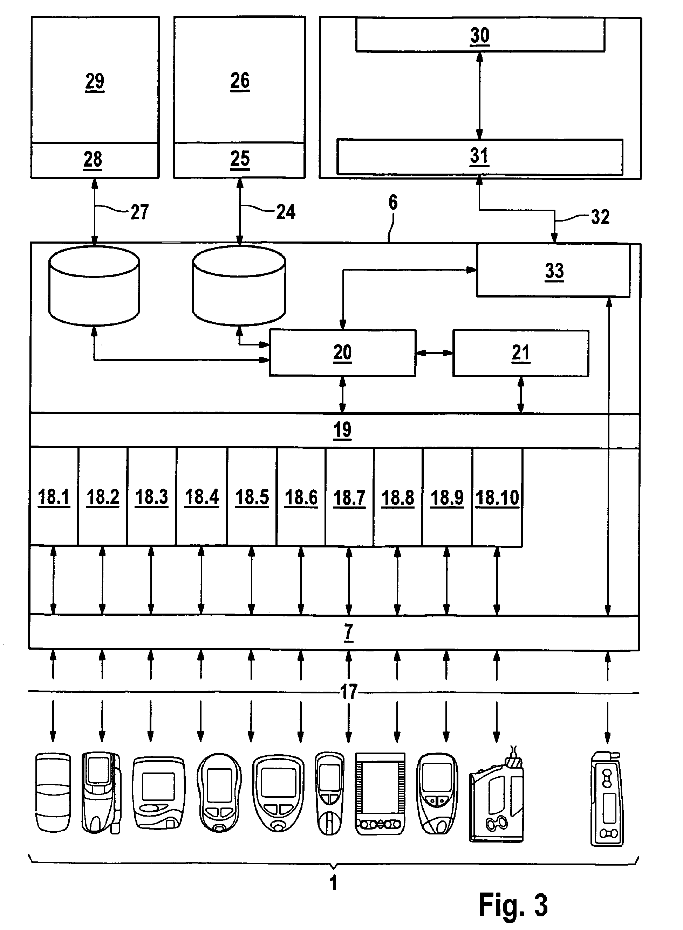

Die Figur 3 zeigt Details einer Implementierung eines Kommunikations-Adapters 6, an den zahlreiche verschiedene Geräte 1 angeschlossen werden können. Die Geräte 1 kommunizieren über gerätespezifische low level Geräteinterface 17 mit der Adapter-Geräte-Schnittstelle 7 des Kommunikations-Adapters 6. Der Kommunikations-Adapter 6 weist eine Mehrzahl von Gerätetreibern auf, die mittels unterschiedlicher Protokolle 18.1 bis 18.10 die geräteangepaßte Kommunikation mit den Geräten 1 ermöglichen. Der universelle Dateimanager 19 steuert die Datenverarbeitung 20 des Prozessors und der Datenspeicher 21 in dem Kommunikations-Adapter 6. Die Datenausgabe erfolgt durch HTML-Berichte 22 oder in Form von XML-Daten 23. Die HTML-Berichte 22 werden über Lesebefehle 24 und den Datenzugriff 25 auf einem Internet-Browser 26 eines Computers 12 dargestellt. Die XML-Daten werden über einen Lesebefehl 27 und einen Datenzugriff 28 anderen PC-Anwendungen, z.B. Excel, des Computers 12 bereitgestellt.

Daneben gibt es noch eine konsolidierte Funktionalität 30 eines Human Interface Device Treibers 31. Dieser erzeugt Lese- und Schreibbefehle 32 in Verbindung mit einem USB-IR-Kommando-Umsetzer 33 für die Hardware-Steuerung und das Ausführen von Befehlen.

### Bezugszeichenliste

- 1: Gerät
- 2: Blutglukosemeter
- 3: Insulinpumpe
- 4: Geräte-Adapter-Schnittstelle
- 5: IR-Datenübertragung
- 6: Kommunikations-Adapter
- 7: Adapater-Geräte-Schnittstelle
- 8: drahtgebundene Datenübertragung
- 9: Adapter-Computer-Schnittstelle
- 10: Übertragungskabel
- 11: Computer-Schnittstelle
- 12: Computer
- 13: Tastatur
- 14: Monitor
- 15: drahtgebundene Datenübertragung
- 16: IR-Datenübertragung
- 17: Geräteinterface
- 18: Protokoll
- 19: universeller Dateimanager
- 20: Datenverarbeitung
- 21: Datenspeicher
- 22: HTML-Bericht
- 23: XML-Daten
- 24: Lesebefehl
- 25: Datenzugriff
- 26: Internet-Browser
- 27: Lesebefehl
- 28: Datenzugriff
- 29: PC-Anwendungen
- 30: konsolidierte Funktionalität

- 31: Human Interface Device Treiber
- 32: Lese- und Schreibbefehl
- 33: Kommandoumsetzer

## Patentansprüche

1. Kommunikations-Adapter (6) zur Verwendung mit einem tragbaren, ambulant betreibbaren medizinischen oder therapeutischen Gerät (1), insbesondere einem Gerät
(1) für die Diagnose oder Behandlung einer Blutglukoseerkrankung, zum Übertragen von Daten zwischen dem medizinischen oder therapeutischen Gerät und einem Computer (12), der zum Anzeigen von Betriebsparametern oder Meßdaten des Geräts (1) und/oder zum Bedienen des Geräts (1) dient,
wobei das medizinische oder therapeutische Gerät (1) umfaßt
- einen Geräteprozessor zum Steuern des Gerätes (1) und
- eine Geräte-Adapter-Schnittstelle (4) zur Kommunikation des Geräteprozessors mit dem Kommunikations-Adapter (6),
und wobei der Kommunikations-Adapter (6) umfaßt
- einen Adapterprozessor zum Steuern des Kommunikations-Adapters (6),
- eine Adapter-Geräte-Schnittstelle (7) zur Kommunikation des Kommunikations-Adapters (6) mit dem Gerät (1),
- eine Adapter-Computer-Schnittstelle (9) zur Kommunikation des Adapterprozessors mit einer Computer-Schnittstelle (11) des Computers (12) und
- einen Gerätetreiber mit zugehörigem Übertragungsprotokoll (18) für die Kommunikation mit dem Gerät (1),
**dadurch gekennzeichnet, daß**
der Kommunikations-Adapter (6) die erforderliche Soft- oder Firmware umfaßt, um die von dem Gerät (1) ausgelesenen Daten unabhängig von dem Computer (12) aufzubereiten und in einem von dem Computer (12) mit standardmäßiger Darstellungs-Software darstellbaren Datenformat über die Adapter-Computer-Schnittstelle (9) an den Computer (12) zu senden.

2. Kommunikations-Adapter (6) zur Verwendung mit einem tragbaren, ambulant betreibbaren medizinischen oder therapeutischen Gerät (1), insbesondere einem Gerät
(1) für die Diagnose oder Behandlung einer Blutglukoseerkrankung, zum Übertragen von Daten zwischen dem medizinischen oder therapeutischen Gerät und einem Computer (12), der zum Anzeigen von Betriebsparametern oder Meßdaten des Geräts (1) und/oder zum Bedienen des Geräts (1) dient,
wobei das medizinische oder therapeutische Gerät (1) umfaßt
- einen Geräteprozessor zum Steuern des Gerätes (1) und
- eine Geräte-Adapter-Schnittstelle (4) zur Kommunikation des Geräteprozessors mit dem Kommunikations-Adapter (6),
und wobei der Kommunikations-Adapter (6) umfaßt
- einen Adapterprozessor zum Steuern des Kommunikations-Adapters (6),
- eine Adapter-Geräte-Schnittstelle (7) zur Kommunikation des Kommunikations-Adapters (6) mit dem Gerät (1),
- eine Adapter-Computer-Schnittstelle (9) zur Kommunikation des Adapterprozessors mit einer Computer-Schnittstelle (11) des Computers (12) und
- einen Gerätetreiber mit zugehörigem Übertragungsprotokoll (18) für die Kommunikation mit dem Gerät (1),
insbesondere ein Kommunikations-Adapter (6) nach Anspruch 1,
**dadurch gekennzeichnet, daß**
die Steuerung des Kommunikations-Adapters (6) von dem Computer (12) aus **dadurch** erfolgt, daß von dem Computer (12) Lesebefehle an den Kommunikations-Adapter (6) gesendet werden, durch die in dem Kommunikations-Adapter (6) Triggerdateien gelesen werden, die von der Soft- oder Firmware des Kommunikations-Adapters (6) als Steuerbefehle interpretiert werden.

3. Kommunikations-Adapter (6) nach einem der vorhergehenden Ansprüche, **dadurch gekennzeichnet, daß** die Adapter-Geräte-Schnittstelle (7) zur Kommunikation des Kommunikations-Adapters (6) mit dem Gerät (1) eine Schnittstelle zur drahtlosen Datenübertragung, insbesondere eine Infrarot-Schnittstelle ist.

4. Kommunikations-Adapter (6) nach einem der vorhergehenden Ansprüche, **dadurch gekennzeichnet, daß** die Adapter-Computer-Schnittstelle (9) zur Kommunikation des Adapterprozessors mit der Computer-Schnittstelle (11) des Computers (12) eine Schnittstelle zur drahtgebundenen Datenübertragung ist.

5. Kommunikations-Adapter (6) nach dem vorhergehenden Anspruch, **dadurch gekennzeichnet, daß** die drahtgebundene Adapter-Computer-Schnittstelle (9) eine serielle Schnittstelle, eine parallele Schnittstelle, eine Firewire-Schnittstelle oder bevorzugt eine USB-Schnittstelle ist.

6. Kommunikations-Adapter (6) nach einem der vorhergehenden Ansprüche, **dadurch gekennzeichnet, daß** er eine Mehrzahl von Gerätetreibern mit zugehörigen Übertragungsprotokollen (18) aufweist, die bei einem über die Adapter-Gerät-Schnittstelle (7) mit dem Kommunikations-Adapter (6) kommunizierenden Gerät (1) automatisch abgefragt werden, um einen für das aktuelle Gerät (1) passenden Gerätetreiber aus der Mehrzahl der Gerätetreiber zu wählen.

7. Kommunikations-Adapter (6) nach einem der vorhergehenden Ansprüche, **dadurch gekennzeichnet, daß** das von dem Kommunikations-Adapter (6) zur Darstellung der mit dem Computer (12) von dem Gerät (1) ausgelesenen Daten erzeugte Datenformat ein von einem Internet-Browser darstellbares Format, vorzugsweise ein HTML-Format ist.

8. Kommunikations-Adapter (6) nach einem der vorhergehenden Ansprüche, **dadurch gekennzeichnet, daß** der Kommunikations-Adapter (6) zusätzlich die von dem Gerät (1) ausgelesenen Daten in einem abspeicherbaren Datenformat erzeugt und diese Daten in mindestens einer Datei in dem Kommunikations-Adapter abspeichert, von wo sie über die Adapter-Computer-Schnittstelle (9) von dem Computer (12) auslesbar sind.

9. Kommunikations-Adapter (6) nach einem der vorhergehenden Ansprüche, **dadurch gekennzeichnet, daß** er keine eigene oder interne Stromquelle aufweist und seine Stromversorgung über die Adapter-Computer-Schnittstelle (9) von dem Computer (12) erfolgt.

10. Kommunikations-Adapter (6) nach einem der vorhergehenden Ansprüche, **dadurch gekennzeichnet, daß** er zum Programmieren des Geräts (1) von dem Computer (12) aus ausgebildet ist, wobei die Programmierung des Geräts (1) von dem Computer (12) über die Computer-Schnittstelle (11) des Computers (12) an den Kommunikations-Adapter (6) und von dem Kommunikations-Adapter (6) über die Adapter-Geräte-Schnittstelle des Kommunikations-Adapters (6) an das Gerät (1) erfolgt.

11. Kommunikations-Adapter (6) nach einem der vorhergehenden Ansprüche, **dadurch gekennzeichnet, daß** das Gerät (1) ein analytisches Meßgerät zum Bestimmen oder Aufzeichnen eines medizinisch bedeutsamen Parameters, insbesondere ein Blutglukosemeter (2), ein Cholesterinmeßgerät, ein Blutgerinnungsparameter-Meßgerät, ein Blutdruckmeßgerät, ein Gerät zum Messen des Körpergewichts, ein Blutglukoserecorder oder ein Blutdruckrecorder, oder ein Gerät zum Injizieren eines medizinischen Wirkstoffs in einen Körper, insbesondere eine Insulinpumpe (3) ist.

12. Verfahren zum Übertragen von Daten zwischen einem tragbaren, ambulant betreibbaren medizinischen oder therapeutischen Gerät (1), insbesondere einem Gerät
(1) für die Diagnose oder Behandlung einer Blutglukoseerkrankung und einem Computer (12), der zum Anzeigen von Betriebsparametern oder Meßdaten des Geräts
(1) und/oder zum Bedienen des Geräts (1) dient, mittels eines Kommunikations-Adapters (6),
wobei das medizinische oder therapeutische Gerät (1) umfaßt
- einen Geräteprozessor, der das Gerät (1) steuert, und
- eine Geräte-Adapter-Schnittstelle (4) zur Kommunikation des Geräteprozessors mit dem Kommunikations-Adapter (6),
und wobei der Kommunikations-Adapter (6) umfaßt
- einen Adapterprozessor, der den Kommunikations-Adapter (6) steuert,
- eine Adapter-Geräte-Schnittstelle (7) zur Kommunikation des Kommunikations-Adapters (6) mit dem Gerät (1),
- eine Adapter-Computer-Schnittstelle (9) zur Kommunikation des Adapterprozessors mit einer Computer-Schnittstelle (11) des Computers (12) und
- einen Gerätetreiber mit zugehörigem Übertragungsprotokoll (18) für die Kommunikation mit dem Gerät (1),
**dadurch gekennzeichnet, daß**
der Kommunikations-Adapter (6) die erforderliche Soft- oder Firmware umfaßt, mit der die von dem Gerät
(1) ausgelesenen Daten unabhängig von dem Computer (12) aufbereitet und in einem von dem Computer (12) mit standardmäßiger Darstellungs-Software darstellbaren Datenformat über die Adapter-Computer-Schnittstelle (9) an den Computer (12) gesendet werden.

13. Verfahren zum Übertragen von Daten zwischen einem tragbaren, ambulant betreibbaren medizinischen oder therapeutischen Gerät (1), insbesondere einem Gerät (1) für die Diagnose oder Behandlung einer Blutglukoseerkrankung und einem Computer (12), der zum Anzeigen von Betriebsparametern oder Meßdaten des Geräts (1) und/oder zum Bedienen des Geräts (1) dient, mittels eines Kommunikations-Adapters (6),
wobei das medizinische oder therapeutische Gerät (1) umfaßt
- einen Geräteprozessor, der das Gerät (1) steuert, und
- eine Geräte-Adapter-Schnittstelle (4) zur Kommunikation des Geräteprozessors mit dem Kommunikations-Adapter (6),
und wobei der Kommunikations-Adapter (6) umfaßt
- einen Adapterprozessor, der den Kommunikations-Adapter (6) steuert,
- eine Adapter-Geräte-Schnittstelle (7) zur Kommunikation des Kommunikations-Adapters (6) mit dem Gerät (1),
- eine Adapter-Computer-Schnittstelle (9) zur Kommunikation des Adapterprozessors mit einer Computer-Schnittstelle (11) des Computers (12) und
- einen Gerätetreiber mit zugehörigem Übertragungsprotokoll (18) für die Kommunikation mit dem Gerät (1),
**dadurch gekennzeichnet, daß**
der Kommunikations-Adapter (6) von dem Computer (12) aus **dadurch** gesteuert wird, daß von dem Computer (12) Lesebefehle an den Kommunikations-Adapter (6) gesendet werden, durch die in dem Kommunikations-Adapter (6) Triggerdateien gelesen werden, die von der Soft- oder Firmware des Kommunikations-Adapters (6) als Steuerbefehle interpretiert werden.

14. Verfahren nach Anspruch 12 oder 13, **dadurch gekennzeichnet, daß** der Kommunikations-Adapter (6) eine Mehrzahl von Gerätetreibern mit zugehörigen Übertragungsprotokollen (18) aufweist, die bei einem über die Adapter-Geräte-Schnittstelle (7) mit dem Kommunikations-Adapter (6) kommunizierenden Gerät (1) automatisch abgefragt werden, um einen für das aktuelle Gerät (1) passenden Gerätetreiber aus der Mehrzahl der Gerätetreiber zu wählen.

15. System zum Betreiben eines tragbaren, ambulant betreibbaren medizinischen oder therapeutischen Geräts (1), insbesondere einem Gerät (1) für die Diagnose oder Behandlung einer Blutglukoseerkrankung, mit einer Datenübertragung zwischen dem medizinischen oder therapeutischen Gerät (1) und einem Computer (12), der zum Anzeigen von Betriebsparametern oder Meßdaten des Geräts (1) und/oder zum Bedienen des Geräts (1) dient, **dadurch gekennzeichnet, daß** es einen Kommunikations-Adapter (6) nach einem der Ansprüche 1 bis 11 und ein medizinisches oder therapeutisches Gerät (1) mit einem Geräteprozessor, der das Gerät (1) steuert und mit einer Geräte-Adapter-Schnittstelle (4) zur Kommunikation des Geräteprozessors mit dem Kommunikations-Adapter (6) umfaßt.
